(19) Europäisches Patentamt
European Patent Office
Office européen des brevets

(11) Veröffentlichungsnummer: **0 004 011**
**B1**

(12) # EUROPÄISCHE PATENTSCHRIFT

(45) Veröffentlichungstag der Patentschrift: **01.04.81**

(21) Anmeldenummer: **79100560.6**

(22) Anmeldetag: **24.02.79**

(51) Int. Cl.³: **C 07 C 143/822,**
A 61 K 31/18,
A 61 K 31/19,
A 61 K 31/085

(54) **Phenoxyalkylcarbonsäure-Derivate, Verfahren zu deren Herstellung sowie diese Verbindungen enthaltende Arzneimittel.**

(30) Priorität: **04.03.78 DE 2809377**

(43) Veröffentlichungstag der Anmeldung:
**19.09.79 Patentblatt 79/19**

(45) Bekanntmachung des Hinweises auf die Patenterteilung:
**01.04.81 Patentblatt 81/13**

(84) Benannte Vertragsstaaten:
**BE CH DE FR GB IT LU NL SE**

(56) Entgegenhaltungen:
**DE - A - 2 149 070**
**DE - A - 2 257 148**
**DE - A - 2 405 622**
**DE - A - 2 541 342**

(73) Patentinhaber: **BOEHRINGER MANNHEIM GMBH**
**Sandhofer Strasse 112-132 Postfach 31 01 20**
**D-6800 Mannheim 31-Waldhof (DE)**

(72) Erfinder: **Witte, Ernst-Christian, Dr.**
**Beethovenstrasse 2**
**D-6800 Mannheim 1 (DE)**
Erfinder: **Wolff, Hans Peter, Dr.**
**Rebenweg 24**
**D-6945 Hirschberg-Grossachsen (DE)**
Erfinder: **Thiel, Max, Dr.**
**S 6, 35**
**D-6800 Mannheim 1 (DE)**
Erfinder: **Stegmeier, Karlheinz, Dr.**
**Kirchbergstrasse 17**
**D-6148 Heppenheim (DE)**
Erfinder: **Roesch, Egon, Dr.**
**Am Oberen Luisenpark 22**
**D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 004 011 B1

Phenoxyalkylcarbonsäure-Derivate, Verfahren zu deren Herstellung sowie diese Verbindungen enthaltende Arzneimittel

Die vorliegende Erfindung betrifft neue Phenoxyalkylcarbonsäure-Derivate, Verfahren zu deren Herstellung sowie diese Verbindungen enthaltende Arzneimittel.

Die neuen Verbindungen sowie ihre pharmakologisch unbedenklichen Salze weisen eine signifikante lipidsenkende Wirkung, im wesentlichen jedoch eine ausgeprägte Hemmwirking auf die Thrombozytenaggregation auf.

In dem DE—BP 21 49 070, der DE—OS 24 05 622 und DE—OS 25 41 342 sind Phenoxyalkylcarbonsäure-Derivate mit lipidsenkender Wirkung beschrieben, deren Phenylgruppe durch Acylaminoalkyl-Reste unterschiedlicher Bedeutung substituiert ist.

Die vorliegende Erfindung betrifft daher Phenoxyalkylcarbonsäure-Derivate der allgemeinen Formel I

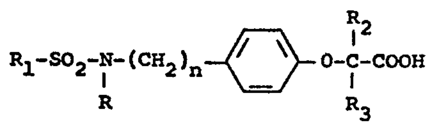
(I)

in welcher

R Wasserstoff oder eine niedere Alkylgruppe,

$R_1$ eine Alkylgruppe oder eine Aryl-, Aralkyl- oder Aralkenylgruppe, deren Aryl-Rest jeweils gegebenenfalls ein- oder mehrfach durch Hydroxyl, Halogen, Trifluoromethyl oder niedere Alkyl-, Alkoxy- oder Acylgruppen substituiert sein kann,

$R_2$ und $R_3$, die gleich oder verschieden sein können, Wasserstoff oder eine niedere Alkylgruppe, und

n die Zahlen 0 bis 3

bedeuten, sowie deren physiologisch unbedenklichen Salze, Ester und Amide.

Die neuen Phenoxyalkylcarbonsäure-Derivate der allgemeinen Formel I, deren Phenylgruppe durch einen Sulfonylamino- oder Sulfonylaminoalkyl-Rest substituiert ist, sowie deren pharmakologisch unbedenklichen Salze, deren Ester und Amide zeigen neben ihrer signifikanten lipidsenkenden Wirkung eine im Vergleich zum nächstliegenden Stand der Technik deutlich bessere Hemmung der induzierten Thrombozytenaggregation.

Zum Beweis hierfür wurde im pharmakologischen Test die Hemmung der durch Adrenalin induzierten Aggregation für (= Acetylsalicylsäure) Handelsprodukt COLFARIT und die Verbindung des Beispiels 1 (=4-[2-(Benzolsulfonylamino)-ethyl]-phenoxyessigsäure) ermittelt. Während das Handelsprodukt bei einer Konzentration von $5 \times 10^{-5}$ mol die Adrenalin-induzierte Aggregation nicht mehr hemmt, zeigt die erfindungsgemässe Substanz bei dieser Konzentration eine 100%ige Hemmwirkung.

Anhand einer geeigneten Auswahl von Verbindungen konnte gezeigt werden, dass auch die übrigen beanspruchten Substanzen ebenfalls eine ausgeprägte Hemmung der induzierten Thrombozytenaggregation bewirken.

Unter der Alkylgruppe des Substituenten $R_1$ sollen geradkettige oder verzweigte Ketten mit 1—16 Kohlenstoffatomen verstanden werden. In diesem Sinne bevorzugt sind der Methyl-, Ethyl-, der Octyl- und der Hexadecylrest.

Als Aralkylreste kommen solche infrage, deren Alkylrest 1—5 Kohlenstoffatome enthält, die geradkettig oder verzweigt sind. Bevorzugt sind der Phenethyl- und der 4-Chlorphenethyl-Rest.

Als Aralkenyl-Reste kommen solche infrage, deren Alkenylrest 2—3 Kohlenstoffatome enthält. Hier sind bevorzugt der Styryl- und der 4-Chlor-styrylrest.

Unter "Arylrest" sind aromatische Kohlenwasserstoffe mit 6—14 C-Atomen, insbesondere der Phenyl-, der Biphenylyl, der Naphthyl- und der Fluorenylrest zu verstehen. Diese Arylreste können in allen möglichen Positionen ein- oder mehrfach substituiert sein, wobei als Substituenten Halogen, Alkyl, Alkoxygruppen, die Hydroxygruppe, Trifluormethyl sowie Acylgruppen infrage kommen.

Unter Halogen verstehe man vorzugsweise Fluor, Chlor und Brom.

Unter "Alkyl" und 'Alkoxygruppen" sind in allen Fällen geradkettige oder verzweigte Reste mit 1—5 Kohlenstoffatomen zu verstehen. Vorzugsweise findet als geradkettiger Alkylrest die Methylgruppe, als verzweigter Alkyrest die tert.- Butylgruppe und als Alkoxygruppe die Methoxygruppe Verwendung. Als bevorzugter Acylrest ist die Acetylgruppe anzusehen.

Die niederen Alkylgruppen R, $R_2$ und $R_3$ können geradkettig oder verzweigt sein und enthalten 1—6, vorzugsweise 1—4 Kohlenstoffatome.

Die von den Carbonsäuren der allgemeinen Formel I abgeleiteten Ester enthalten als Alkoholcomponente niedere einwertige Alkohole, von denen Methanol, Ethanol und Propanol bevorzugt sind, sowie mehrwertige Alkohole, z.B. Glykol oder Glycerin, oder Alkohole mit anderen funktionellen Gruppen, wie z.B. Ethanolamin oder Glykolether.

Die erfindungsgemässen, von den Carbonsäuren der allgemeinen Formel I abgeleiteten Amide enthalten als Aminkomponente z.B. Ammoniak, p-Aminobenzoesäure, $\beta$-Alanin, Ethanolamin und 2-Amino-propanol sowie die Aminosäure

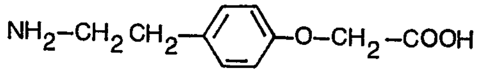 ,

wobei die bis hierher genannten bevorzugt sein sollen, Es kommen aber auch Alkylamine wie z.B. Isopropylamin oder tert.-Butylamin, Dialkylamine wie Diethylamin sowie cyclische Amine wie z.B. Morpholin oder 4-Alkyl- bzw. -Aralkyl- bzw. -Arylpiperazine, z.B. 4-Methylpiperazin, 4-(4-Chlorbenzyl)-piperazin oder 4-(3-Methoxy-phenyl)-piperazin infrage.

Die obige Definition der erfindungsgemässen Verbindungen soll auch alle möglichen Stereoisomeren sowie ihre Mischungen umfassen.

Die Herstellung der Verbindungen der allgemeinen Formel I ist dadurch gekennzeichnet, dass man ein Amin der allgemeinen Formel II

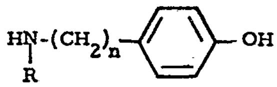 (II),

in welcher R und n die oben angegebene Bedeutung haben, gegebenenfalls unter intermediärem Schutz der Amino- bzw. Hydroxylgruppe in an sich bekannter Weise in beliebiger Reihenfolge mit einer Sulfonsäure der allgemeinen Formel III

$$R_1—SO_2OH \qquad (III),$$

in welcher $R_1$ die oben angegebene Bedeutung hat, bzw. einem Derivat derselben und mit einer Verbindung der allgemeinen Formel IV

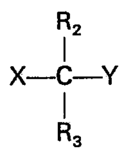 (IV),

umsetzt.

In Formel IV haben $R_2$ und $R_3$ die oben angegebene Bedeutung, X stellt eine reaktive Gruppe dar, und unter Y ist die Gruppe $—COOR_4$ (wobei $R_4$ ein Wasserstoffatom oder eine niedere Alkylgruppe darstellt) oder eine Säureamidgruppe zu verstehen. Y kann aber auch einen Rest darstellen, der nach erfolgter Kondensation in eine Säureamidgruppe oder in die $—COOR_4$-Gruppe überführt wird, worauf man gegebenenfalls einen bestimmten Substituenten $R_4$ anschliessend an die Kondensation in an sich bekannter Weise in einen anderen Substituenten $R_4$ umwandelt und die erhaltenen Verbindungen in pharmakologisch unbedenkliche Salze überführt.

Für den Fall, dass $R_2$ und $R_3$ niedere Alkylgruppen bedeuten, können die Phenole der allgemeinen Formel II oder deren Reaktionsprodukte mit einer Sulfonsäure der allgemeinen Formel III auch mit einem Gemisch aus einem aliphatischen Keton, Chloroform und einem Alkalihydroxid umgesetzt werden. Vorzugsweise wird diese Variante zur Herstellung von Isobuttersäure-Derivaten verwendet, wobei als Keton Aceton eingesetzt wird (vgl. hierzu Gazz. Chim. ital. 77 (1947), S. 431).

Das erfindungsgemässe Verfahren führt man zweckmässig in zwei Stufen durch. Die Kondensation der Verbindungen der allgemeinen Formel II mit Sulfonsäuren III oder deren Derivaten einerseits und Verbindungen der allgemeinen Formel IV andererseits wird vorzugsweise so durchgeführt, dass man zunächst eine der beiden reaktiven Gruppen der Verbindung II mit einer leicht abspaltbaren Schutzgruppe blockiert, die erhaltene Verbindung mit einer Sulfonsäure III oder einem Derivat davon bzw. mit einer Verbindung der allgemeinen Formel IV umsetzt, die Schutzgruppe wieder abspaltet und anschliessend dieses reaktive Zwischenprodukt mit der noch nicht eingesetzten Verbindung der allgemeinen Formel IV bzw. III umsetzt. Bevorzugt wird ein Verfahrensweg, bei dem die an der Aminogruppe geschützte Verbindung II zunächst mit einer Verbindung IV zur Umsetzung gebracht wird. Nach Abspaltung der Schutzgruppe erfolgt dann die Reaktion mit einer Sulfonsäure III bzw. mit einem ihrer Derivate.

Anstelle der freien Amine II kann man auch deren Salze einsetzen.

Eine weitere Möglichkeit zur Herstellung von Verbindungen der allgemeinen Formel I, ihrer Salze sowie ihrer Ester und Amide besteht darin, dass man ein Sulfonamid der allgemeinen Formel V

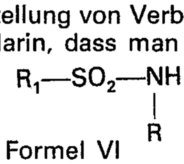 (V),

mit einer Verbindung der allgemeinen Formel VI

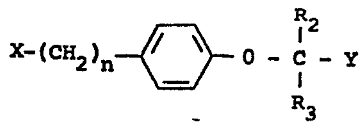

(VI)

zu der Verbindung VII

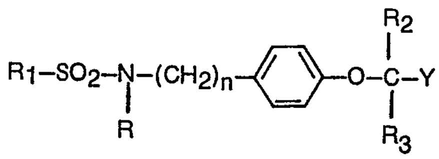

(VII),

umsetzt.

Die in den Formeln, V, VI und VII verwendeten Symbole R, $R_1$ X, n, $R_2$, $R_3$ und Y haben die oben angegebene Bedeutung.

Eine dritte Synthesemöglichkeit besteht in einer Umacylierung:

Setzt man eine freie Sulfonsäure III mit einer Verbindung der allgemeinen Formel VIII

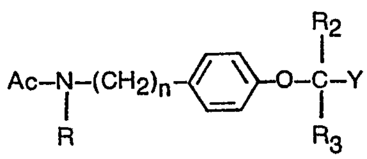

(VIII),

in welcher n, R, $R_2$, $R_3$ und Y die oben angegebene Bedeutung haben und Ac einen leicht austauschbaren Acylrest darstellt, in einem geeigneten Lösungsmittel um, so erhält man ebenfalls ein Sulfonamid der allgemeinen Formel VII.

Als reaktive Derivate der Sulfonsäuren III kommen insbesondere die Halogenide sowie die Ester infrage. Die Umsetzungen der Sulfonsäurehalogenide mit Verbindungen der allgemeinen Formel II erfolgen zweckmässig unter Zusatz eines säurebindenden Mittels, wie z.B. Alkaliacetat, Natriumhydrogencarbonat, Natriumcarbonat, Natriumphosphat, Calciumoxid, Calciumcarbonat oder Magnesiumcarbonat. Diese Funktion können aber auch organische Basen wie z.B. Pyridin oder Triethylamin übernehmen, wobei als inertes Lösungsmittel z.B. Ether, Benzol, Methylenchlorid, Dioxan oder ein Ueberschuss des tertiären Amins dient.

Bei Einsatz anorganischer Säurebinder verwendet man als Reaktionsmedium z.B. Wasser, wässriges Ethanol oder wässriges Dioxan.

Für eine Umsetzung der Verbinding II mit der Verbindung IV hat es sich als vorteilhaft herausgestellt, zunächst die Aminogruppe der Verbinding II in eine geschützte Gruppe, z.B. die Phthalimidgruppe, zu überführen, die nach der Umsetzung z.B. mit Hydroxylamin in an sich bekannter Weise leicht wieder gespalten werden kann. Es können auch andere aus der Peptidchemie bekannte Gruppen zum Schutz der Aminogruppe eingeführt und nach der Umsetzung wieder abgespalten werden. Bevorzugt ist die Blockierung der Aminogruppe durch eine Acylgruppe, wie die Formyl- oder Acetylgruppe, die nach der Umsetzung mit starken Basen, wie z.B. Natriumhydroxid oder Kaliumhydroxid, aber auch mit wässrigen Mineralsäuren, wie z.B. Salzsäure, leicht wieder abgespalten werden kann.

Als reaktive Verbindung IV kommt insbesondere diejenige infrage, bei der X das Anion einer starken Säure, z.B. einer Halogenwasserstoff- oder Sulfonsäure darstellt. Die Reaktion kann weiterhin begünstigt werden, indem man die phenolische Hydroxylgruppe der Verbindung II z.B. durch Umsetzung mit Natriumalkoholat in ein Phenolat überführt. Die Reaktion der beiden Komponenten wird in Lösungsmitteln, wie z.B. Toluol oder Xylol, Methylethylketon oder Dimethylformamid, vorzugsweise in der Wärme durchgeführt.

Zur Alkylierung der Sulfonsäureamide V verwendet man bevorzugt Verbindungen VI, in denen X eine Arylsulfonyloxygruppe darstellt. Bevorzugt soll X eine Toluolsulfonyloxygruppe bedeuten. Als Alkylierungsmittel dienen also bevorzugt Arylsulfonsäurealkylester, eine Methode, die in ihrer Anwendung auf Sulfonsäureamide z.B. bei Klamann et al., Monatshefte für Chemie Bd. *83* (1952), S. 871, beschrieben ist. Die Umsetzungen erfolgen in alkalischem Milieu, bevorzugt ist als Reaktionsmedium heisse, konzentrierte Sodalösung.

Die Umacylierungsreaktion zwischen einer freien Sulfonsäure III und einem Acylamin VIII wird unter einsatz bevorzugt äquimolarer Mengen beider Reaktionspartner in einem polaren Lösungsmittel vorgenommen. Als solche polaren Lösungsmittel dienen z.B. Alkohole, insbesondere Ethanol und Methanol. Die Umsetzung verläuft bevorzugt bei der Siedetemperatur dieser Lösngsmittel. Als leicht austauschbarer Acylrest ist z.B. der Acetylrest zu nennen.

Als Substituenten Y der allgemeinen Formel IV, die in die $-COOR_4$-Gruppe überführt werden

können, kommen beispielsweise die Nitril-, Carbaldehyd-, Hydroxymethyl-, Aminomethyl- und Formylgruppe infrage.

Die gegebenenfalls nachträgliche N-Alkylierung einer Verbinding der allgemeinen Formel I, in denen R = Wasserstoff bedeutet, kann nach bekannten Methoden durch geführt werden, vorzugsweise indem man eine Verbinding mit R = Wasserstoff mit einem Alkylhalogenid oder einem Dialkylsulfat in Gegenwart eines säurebindenden Mittels, wie z.B. Natriumhydroxid, umsetzt.

Die gegebenenfalls im Anschluss an die Kondensation durchzuführende Umwandlung des Substituenten $R_4$ erfolgt beispielsweise durch Verseifung der Carbonsäureester ($R_4$ = Alkyl) zu den entsprechenden Carbonsäuren ($R_4$ = Wasserstoff) mit Mineralsäuren oder Alkalihydroxiden in einem polaren Lösungsmittel (wie Wasser, Methanol, Ethanol, Dioxan oder Aceton). Vorteilhaft wird die Verseifung mit einer starken Base (wie Natrium- oder Kaliumhydroxid) in einem Gemisch aus Methanol und Wasser bei Raumtemperatur oder bei mässig erhöhten Temperaturen durchgeführt. Umgekehrt kann man aber auch die Carbonsäuren in üblicher Weise verestern oder Ester mit einem bestimmten Rest $R_4$ durch Umestern in einen Ester mit einem anderen Rest $R_4$ umwandeln. Die Veresterung der Carbonsäuren wird zweckmässig in Gegenwart eines sauren Katalysators, wie z.B. Chlorwasserstoff, Schwefelsäure, p-Toluolsulfonsäure oder eines stark sauren Ionenaustauschharzes, vorgenommen. Umesterungen hingegen erfordern den Zusatz einer geringen Menge einer basischen Substanz, z.B. eines Alkali- oder Erdalkalihydroxids oder eines Alkalialkoholats Für die Veresterung der Carboxylgruppe bzw. für eine Umesterung eignen sich prinzipiell alle Alkohole. Bevorzugt sind die niederen einwertigen Alkohole wie Methanol, Ethanol oder Propanol, sowie mehrwertige Alkohole, z.B. Glykol, oder Alkohole mit anderen funktionellen Gruppen, wie Ethanolamin oder Glykolether.

Die erfindungsgemässen, von den Carbonsäuren der allgemeinen Formel I abgeleiteten Amide werden bevorzugt nach an sich bekannten Methoden aus den Carbonsäuren oder ihren reaktiven Derivaten (wie z.B. Carbonsäurehalogeniden, -estern, -aziden, -anhydriden oder gemischten Anhydriden) durch Umsetzung mit Aminen hergestellt. Als Aminokomponenten kommen z.B. Ammoniak, Alkylamine, Dialkylamine, aber auch Aminoalkohole wie z.B. Ethanolamin und 2-Aminopropanol sowie Aminosäuren wie z.B. p-Aminobenzoesäure, β-Alanin und andere infrage. Andere wertvolle Aminkomponenten sind Alkyl-, Aralkyl- und Arylpiperazine.

Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süssstoffe enthalten.

Die nachfolgenden Beispiele zeigen einige der zahlreichen Verfahrensvarianten, die zur Synthese der erfindungsgemässen Verbindungen verwendet werden können. Sie sollen jedoch keine einschränkung des Erfindungsgegenstandes darstellen.

### Beispiel 1

*4-[2-(Benzolsulfonylamino)-ethyl]-phenoxyessigsäure*

*Verfahren I*

a) Man erhitzt ein Gemisch aus 240 g (1.34 mol) N-Acetyltyramin, 370 g (2.68 mol) wasserfreiem, pulverisiertem Kaliumcarbonat und 2.5 ltr. 2-Butanon unter Rühren 2 Stunden auf Rückflußtemperatur, setzt dann 266 g (1.47 mol) Bromessigsäureethylester und 1.5 g Kaliumiodid zu und erhitzt wiederum auf Rückflußtemperatur. Nach etwa 6 Stunden ist die Umsetzung vollständig. Man filtriert das Reaktionsgemisch, wäscht den Filterkuchen mit Aceton und dampft die vereinigten Filtrate im Vakuum ein. Der Rückstand wird in 1.75 ltr. Methylenchlorid aufgenommen. Mas wäscht die Methylenchloridphase dreimal mit je 300 ml 0.5 N Natronlauge und einmal mit 300 ml Wasser, trocknet mittels Natriumsulfat und dampft im Vakuum ein. Man erhält so 321 g (98% d. Th.) 4-(2-Acetylaminoethyl)-phenoxyessigsäureethylester mit dem Schmp. 85°C.

Auf dem gleichen Verfahrensweg läßt sich 4-(2-Acetylaminoethyl)-phenoxyessigsäureethylester

auch aus N-Acetyltyramin und *Chlor*essigsäureethylester herstellen, Reaktionszeit 9 Stunden, Ausbeute quantitativ, Schmp. 83—84.5°C.

b) Man hält ein Gemisch aus 489.6 g (1.845 mol) 4-(2-Acetylaminoethyl)-phenoxyessigsäureethylester und 2.77 ltr. (5.54 mol) 2 N Salzsäure unter Rühren 8 Stunden auf Rückflußtemperatur, kühlt dann ab und bringt die Lösung mit etwa 460 ml 10 N Natronlauge auf pH 6. Nach Abkühlen im Eisbad wird abgesaugt. Man behandelt den Filterkuchen zweimal mit je 250 ml Wasser, saugt scharf ab und trocknet bei 50°C im Vakuum. Es resultieren 299.5 g (83% d. Th.) 4-(2-Aminoethyl)-phenoxyessigsäure mit dem Schmp. 292°C (Zers.)

c) Man suspendiert 280 g (1.435 mol) 4-(2-Aminoethyl)-phenoxyessigsäure in einer Lösung aus 2.85 ltr. Wasser und 436 g (3.157 mol) wasserfreiem Kaliumcarbonat und läßt unter Rühren innerhalb 45 Minuted 266 g (1.507 mol) Benzolsulfochlorid zulaufen. Man rührt anschließend 2.5 Stunden bei 80°C. Nach dem Erkalten gibt man zum Reaktionsgemisch 1 ltr. Essigsäureethylester und säuert unter Rühren mit 800 ml 6 N Salzsäure an. Dann trennt man die organische Phase ab, extrahiert die wässrige Phase mit 1 ltr. Essigsäureethylester, vereinigt die organischen Phasen und extrahiert sie nun mit einer für die Salzbildung ausreichenden Menge gesättigter Natriumhydrogencarbonatlösung. Man filtriert die wässrige Phase und bringt sie unter Rühren mit 6 N Salzsäure auf pH 1, wobei das Produkt in Form eines bräunlichen Granulats ausfällt. Dieses wird abgesaugt, mit etwas Wasser gewaschen, dann an der Luft getrocknet. Man löst die Substanz nun in ca. 3 ltr. warmem Ether. Dabei bleibt ein schmieriger, dunkelbrauner Rückstand ungelöst. Die klare Etherphase wird abgegossen und eingedampft, wobei 312 g (65% d. Th.) farblose 4-[2-(Benzolsulfonylamino)-ethyl]-phenoxyessigsäure mit dem Schmp. 117—118°C resultieren. Schmp. nach Umkristallisieren aus Toluol: 119—120°C.

Zur Herstellung des als Ausgangsprodukt verwendeten N-Acetyltyramins kann man die folgenden beiden Methoden benutzen:

1. Man versetzt unter Rühren 64.0 g (0.466 mol) Tyramin mit 200 ml Acetanhydrid, wobei unter spontaner Erwärmung eine klare Lösung entsteht. Diese Lösung wird mit einigen Kristallen N-Acetyltyramin angeimpft, wonach sofortige Kristallisation eintritt. Man kühlt schnell ab, saugt ab, wäscht mit Ether und Wasser und trocknet. Ausbeute 59 g (71% d. Th.) N-Acetyltyramin vom Schmp. 124—126°C. Durch Eindampfen der Mutterlauge, Lösen des Rückstandes in verdünnter Natronlauge, Filtrieren und Ansäuern des Filtrats erhält man weitere 5.5 g (6% d. Th.) vom Schmp. 122—124°C. Aus Essigester umkristallisiert schmilzt das N-Acetyltyramin bei 129—131°C.

2. Zu einer Lösung aus 54.9 g (0.4 mol) Tyramin und 200 ml Pyridin werden unter Rühren bei 30—35°C 65.8 g (0.84 mol) Acetylchlorid zugetropft. Anschließend erhitzt man 15 Minuten auf dem siedenden Wasserbad, kühlt dann ab und gießt in ein Eis-Wasser-Gemisch. Durch Zugabe von konzentrierter Salzsäure wird deutlich sauer gemacht, anschließend wird mit Chloroform extrahiert. Die Chloroformphase wird mit Wasser gewaschen, über Calciumchlorid getrocknet und dann eingedampft. Es bleibt ein Rückstand von 88.5 g (quant. Ausbeute) Diacetyltyramin vom Schmp. 99—100°C (aus Benzol). Das Diacetyltyramin wird nun in 500 ml Methanol gelöst. Man tropft 800 ml (0.8 mol) 1 N Kalilauge zu (dabei steigt die Temperatur auf ca. 30°C) und hält anschließend 2 Stunden auf 50°C Innentemperatur. Dann wird abgekühlt, mit konzentrierter Salzsäure schwach angesäuert und das Methanol im Vakuum agbedampft. Das auskristallisierte Produkt wird abgesaugt, gründlich mit Wasser gewaschen, dann getrocknet. Ausbeute 58.3 g (81% d. Th.) Schmp. 131°C (aus Essigester).

*Verfahren II*

Zu einem Gemisch aus 15.0 g (0.11 mol) Tyramin, 18.0 g (0.22 mol) wasserfreiem Natriumacetat und 250 ml 97-proz. Ethanol tropft man 19.3 g (0.11 mol) Benzolsulfonylchlorid und erhitzt anschließend 2 Stunden auf Rückflußtemperatur. Dann wird im Vakuum vom Ethanol befreit, der Rückstand wird mit Wasser versetzt und die wässrige Phase mit 2 N Salzsäure angesäuert. Man nimmt das ausfallende Produkt in Ether auf, extrahiert noch mehrmals mit Ether und trocknet die vereinigten Etherphasen mit Natriumsulfat. Dann wird der Ether abdestilliert und der Rückstand mit Ligroin verrieben. Zur Reinigung löst man in 2 N Natronlauge, behandelt die Lösung mit Aktivkohle und fällt das Phenol mit verdünnter Salzsäure wieder aus. Nach Auswaschen mit Wasser und Trocknen erhält man 23.5 g (77% d. Th.) 4-[2-(Benzolsulfonylamino)-ethyl]-phenol mit dem Schmp. 131°C.

In der siedehitze tropft man zu einem Gemisch aus 18.0 g (65 mmol) 4-[2-(Benzolsulfonylamino)-ethyl]-phenol, 8.95 g (65 mmol) wasserfreiem, pulverisiertem Kaliumcarbonat und 250 ml Ethanol unter Rühren 11.9 g (71.5 mmol) Bromessigsäureethylester und hält weitere 2.5 Stunden auf Rückflußtemperatur. Dann dampft man das Reaktionsgemisch im Vakuum ein, extrahiert den Rückstand mehrmals mit Ether und dampft die Etherextrakte ein. Das zurückbleibende Produkt wird aus einem Gemisch aus Essigsäureethylester und Ligroin umkristallisiert. Es resultieren 9.8 g (42% d. Th.) 4-[2-(Benzolsulfonylamino)-ethyl]-phenoxyessigsäureethylester mit dem Schmp. 68—70°C.

Man hält ein Gemisch aus 22.1 g (61 mmol) 4-[2-(Benzolsulfonylamino)-ethyl]-phenoxyessigsäureethylester, 183 ml (183 mmol) 1 N Kalilauge und 250 ml Methanol 2 Stunden bei 35°C. Dann wird mit 2 N Salzsäure angesäuert. Man dampft das Methanol ab und extrahiert die zurückbleibende wässrige Phase mehrmals mit Methylenchlorid. Die vereinigten Methylenchloridphasen werden mit Wasser gewaschen, über Natriumsulfat getrocknet und eingedampft. Man verreibt den Eindampfrückstand mit Ligroin und saugt ab. Es resultieren 17.6 g (86% d. Th.) 4-[2-(Benzolsulfonylamino)-ethyl]-phenoxyessigsäure mit dem Schmp. 116—118°C.

*Verfahren III*

Zu einer eisgekühlten Lösung aus 19.5 g (75 mmol) 4-(2-Aminoethyl)-phenoxyessigsäureethylesterhydrochlorid in 250 ml abs. Pyridin werden im Laufe einer Stunde unter Rühren 13.3 g (75 mmol) Benzolsulfochlorid zugetropft. Dann entfernt man das Kühlbad und rührt 2 Stunden bei Raumtemperatur. Anschließend gießt man in Eiswasser und säuert mit konzentrierter Salzsäure an, wobei sich ein Öl abscheidet, welches in Ether aufgenommen wird. Man extrahiert die wässrige Phase noch mehrmals mit Ether, trocknet die vereinigten Etherphasen mit Natriumsulfat und dampft schließlich ein. Es bleiben 22.3 g (82% d. Th.) 4-[2-(Benzolsulfonylamino)-ethyl]-phenoxyessigsäureethylester mit dem Schmp. 76—77°C.

Zur Verseifung dieses Ethylesters zur 4-[2-(Benzolsulfonylamino)-ethyl]-phenoxyessigsäure siehe oben.

a) *4-[2-(Benzolsulfonylamino)-ethyl]-phenoxyessigsäure-isopropylester*

Man erhitzt ein Gemisch aus 7.28 g (20 mmol) 4-[2-(Benzolsulfonylamino)-ethyl]-phenoxyessigsäureethylester, 75 ml Isopropanol und etwa 50 mg Natriumisopropylat 12 Stunden auf Rückflußtemperatur und destilliert anschließend das Isopropanol ab. Der Rückstand wird mit 0.5 N Salzsäure und Ether behandelt, die Etherphase getrocknet und eingedampft. Man verreibt das zurückbleibende Öl mit Ligroin und erhält 4.5 g (67% d. Th.) 4-[2-(Benzolsulfonylamino)-ethyl]-phenoxyessigsäure-isopropylester mit dem Schmp. 56—57°C.

Beispiel 2

*4-[2-(4-Methoxybenzolsulfonylamino)-ethyl]-phenoxyessigsäure*

Zu einer Lösung aus 11.2 g (50 mmol) 4-(2-Aminoethyl)-phenoxyessigsäureethylester in 125 ml abs. Pyridin tropft man bei 0—10°C in 5 Minuten ein Gemisch aus 10.35 g (50 mmol) 4-Methoxybenzolsulfonylchlorid und 50 ml Pyridin, läßt auf Raumtemperatur kommen und hält dann 45 Minuten auf 60°C. Man dampft anschließend im Vakuum bis zum halben Volumen ein, gießt in Eiswasser und säuert mit Salzsäure an. Die ausfallende viskose Masse wird in Toluol gelöst und die Toluolphase nacheinander mit verd. Salzsäure, Natriumhydrogencarbonatlösung und Wasser extrahiert. Nach Trocknen über $CaCl_2$ dampft man im Vakuum ein und verreibt den Rückstand mit etwas Ether, wobei Kristallisation eintritt. Das Produkt wird abgesaugt und aus sehr wenig Ether umkristallisiert. Man erhält so 13.0 g (66% d. Th.) 4-[2-(4-Methoxybenzolsulfonylamino)-ethyl]-phenoxyessigsäureethylester mit dem Schmp. 82—83°C.

Bei Vorhandensein der 4-[2-(4-Methoxybenzolsulfonylamino)-ethyl]-phenoxyessigsäure kann deren Ethylester auch auf folgendem Wege synthetisiert werden:

Man löst die Säure in der zwanzigfachen Gewichtsmenge absolutem Ethanol und begast von der Oberfläche her mit trockenem Chlorwasserstoff, wobei die Temperatur auf 50—60°C gehalten wird, bis nahe zur Sättigung. Dann wird weitere 30 Minuten bei 50—60°C gehalten, anschließend im Vakuum eingedampft und der gelbliche, zunächst ölige Rückstand aus Ether umkristallisiert. Ausbeute ca. 90% d. Th.

Zu einer Lösung aus 11.8 g (30 mmol) 4-[2-(4-Methoxybenzolsulfonylamino)-ethyl]-phenoxyessigsäureethylester und 70 ml Ethanol tropft man 70 ml (70 mmol) 1 N Kalilauge und hält anschließend 5 Stunden bei 35—40°C. Dann wird im Vakuum das Ethanol abdestilliert und die wässrige Phase mit Ether extrahiert. Zugabe von 35 ml 2 N Salzsäure führt zur Abscheidung eines farblosen Niederschlages, der abgesaugt und aus verd. Essigsäure umkristallisiert wird. Ausbeute 8.66 g (79% d. Th.) 4-[2-(4-Methoxybenzolsulfonylamino)-ethyl]-phenoxyessigsäure mit dem Schmp. 103°C.

Die Darstellung des für die Umsetzung benötigten 4-(2-Aminoethyl)-phenoxyessigsäureethylesters bzw. seines Hydrochlorides kann auf zwei Wegen erfolgen:

A. aus 4-(2-Aminoethyl)-phenoxyessigsäure und Ethanol

Auf eine gekühlte (5—10°C) Lösung aus 67.2 g 4-(2-Aminoethyl)-phenoxyessigsäure und 672 ml abs. Ethanol leitet man unter Rühren trockenes HCl-Gas bis zur Sättigung. Nach Stehen über Nacht wird bei 30°C eingedampft. Den kristallinen Rückstand läßt man zur Trocknung zunächst an der Luft stehen, dann wird über Kaliumhydroxid getrocknet. Ausbeute 85.6 g (96% d. Th.) 4-(2-Aminoethyl)-phenoxyessigsäureethylesterhydrochlorid, Schmp. 158—160°C (Isopropanol). Die freie Base, ein viskoses Öl, kondensiert sehr schnell!

B. über 4-[2-(Phthalimido)-ethyl]-phenol

Man erhitzt ein Gemisch aus 137.1 g (1 mol) Tyramin, 148.1 g (1 mol) Phthalsäureanhydrid, 13 ml Triethylamin und 2 ltr. Toluol unter Rückfluß am Wasserabscheider, bis die theoretische Menge Wasser abgeschieden ist. Dann läßt man erkalten und filtriert den Niederschlag ab. Man erhält 259 g (97% d. Th.) 4-[2-(Phthalimido)-ethyl]-phenol, Fp. 223—226°C. Die Substanz schmilzt nach Umkristallisieren aus Isopropanol bei 228—230°C.

34.7 g (0.13 mol) 4-[2-(Phthalimido)-ethyl]-phenol und 35.9 g (0.26 mol) wasserfreies, pulverisiertes Kaliumcarbonat werden in 300 ml trockenem Aceton 2 Stunden unter Rühren auf Rückflußtemperatur erhitzt. Dann setzt man 31.8 g (0.19 mol) Bromessigsäureethylester und 0.2 g Kaliumiodid zu und halt wiederum 8 Stunden auf Rückflußtemperatur. Man filtriert den anorganischen Niederschlag ab, wäscht mit Aceton nach und dampft die vereinigten Filtrate im Vakuum ein. Der Rückstand wird in Chloroform aufgenommen, die Lösung mit 0.5 N Natronlauge und Wasser gewaschen, getrocknet und eingedampft. Den Rückstand kristallisiert man aus Isopropanol um; Ausbeute 38.8 g (83% d. Th.) 4-[2-(Phthalimido)-ethyl]-phenoxyessigsäureethylester, Fp. 104—106°C.

35.3 g (0.1 mol) dieses Esters werden in 1 ltr. siedendem Ethanol gelöst und heiß mit 7.5 g (0.15 mol) Hydrazinhydrat versetzt. Man läßt die Lösung über Nacht stehen, stellt mit wenig konz. Salzsäure sauer und dampft ein. Den Rückstand rührt man in 1 ltr. Wasser auf, filtriert das Unlösliche ab und dampft das wässrige Filtrat ein. Der Eindampfrückstand wird unter Zusatz von Kohle aus Isopropanol umkristallisiert. Man erhält 17.2 g (66% d. Th.) 4-(2-Aminoethyl)-phenoxyessigsäureethylester-hydrochlorid, Fp. 157—160°C.

Beispiel 3

*4-[2-(4-Fluorbenzolsulfonylamino)-ethyl]-phenoxyessigsäure*

In ein Gemisch aus 160 ml abs. Pyridin, 11.1 g (80 mmol) pulverisiertem, wasserfreiem Kaliumcarbonat und 20.8 g (80 mmol) 4-(2-Aminoethyl)-phenoxyessigsäureethylester-hydrochlorid tropft man bei 10—15°C 16.35 g (84 mmol) 4-Fluorbenzolsulfonylchlorid. Anschließend wird 30 Minuten bei 20°C, dann 5 Minuten bei 80°C gerührt, danach abgekühlt und in Eiswasser gegossen. Man säuert mit konzentrierter Salzsäure an und extrahiert die ausgefallene Substanz mit Methylenchlorid. Nach Trocknen über Natriumsulfat wird die Methylenchloridphase eingedampft und der Eindampfrückstand aus einem Methanol-Wasser-Gemisch umkristallisiert. Man erhält so 25.5 g (84% d. Th.) 4-[2-(4-Fluorbenzolsulfonylamino)-ethyl]-phenoxyessigsäureethylester mit dem Schmp. 75—78°C.

Durch Hydrolyse des Ethylesters in Analogie zu Beispiel 2 erhält man 87% d. Th. 4-[2-(4-Fluorbenzolsulfonylamino)-ethyl]-phenoxyessigsäure mit dem Schmp. 206—208°C.

Zur Herstellung des Natriumsalzes suspendiert man 7.46 g (21.1 mmol) der Säure in 150 ml Methanol, erwärmt auf ca. 40°C und setzt 21.1 ml 1 N Natronlauge zu. Danach wird im Vakuum eingedampft, der Rückstand mit Aceton verrieben, abgesaugt und mit Aceton gewaschen. Ausbeute 7.8 g, Schmp. 260—270°C (Zers.).

In analoger Weise erhält man durch Umsetzen von 4-(2-Aminoethyl)-phenoxyessigsäureethylester-hydrochlorid mit den entsprechenden Sulfonylchloriden in Gegenwart von Pyridin und Kaliumcarbonat:

a) 4-[2-(Methansulfonylamino)-ethyl]-phenoxyessigsäureethylester
als viskoses Öl; Ausbeute 65% d. Th.
IR-Maxima: 3290 $cm^{-1}$, 1750 $cm^{-1}$, 1608 $cm^{-1}$,
und daraus durch Hydrolyse

4-[2-(Methansulfonylamino)-ethyl]-phenoxyessigsäure
Schmp. 142—143°C; Ausbeute 77% d. Th.
Natriumsalz: Schmp. 330°C (Zers.)

b) 4-[2-(p-Toluolsulfonylamino)-ethyl]-phenoxyessigsäureethylester
Schmp. 66—67°C (Ethanol); Ausbeute 74% d. Th.
und daraus durch Hydrolyse

4-[2-(p-Toluolsulfonylamino)-ethyl]-phenoxyessigsäure
Schmp. 119—120°C; Ausbeute 93% d. Th.
Natriumsalz als Monohydrat Schmp. 245—247°C.

c) 4-[2-(1-Naphthalinsulfonylamino)-ethyl]-phenoxyessigsäureethylester
Schmp. 105—106°C (Ethanol); Ausbeute 65% d. Th.
und daraus durch Hydrolyse

4-[2-(1-Naphthalinsulfonylamino)-ethyl]-phenoxyessigsäure
Schmp. 119—120°C (Essigester + Ligroin); Ausbeute 92% d. Th.;
Natriumsalz: Schmp. 238—239°C.

d) 4-[2-(Styrolsulfonylamino)-ethyl]-phenoxyessigsäureethylester
Schmp. 62—63°C (Essigester + Ligroin); Ausbeute 71% d. Th.
und daraus durch Hydrolyse

4-[2-(Styrolsulfonylamino)-ethyl]-phenoxyessigsäure
Schmp. 141—142°C (Aceton + Wasser); Ausbeute 85% d. Th.

e) 4-[2-(4-Chlorstyrolsulfonylamino)-ethyl]-phenoxyessigsäureethylester
Schmp. 91—92°C (Essigester + Ligroin); Ausbeute 76% d. Th.
und daraus durch Hydrolyse

4-[2-(4-Chlorstyrolsulfonylamino)-ethyl]-phenoxyessigsäure
Schmp. 147—148°C (Essigester + Ligroin); Ausbeute 69% d. Th.

f) 4-[2-(4-Chlorbenzolsulfonylamino)-ethyl]-phenoxyessigsäureethylester
Schmp. 61—62°C (Ether); Ausbeute 66% d. Th.
und daraus durch Hydrolyse

4-[2-(4-Chlorbenzolsulfonylamino)-ethyl]-phenoxyessigsäure
Schmp. 127—128°C (Aceton + Wasser); Ausbeute 62% d. Th.

Beispiel 4

*4-(Benzolsulfonylaminomethyl)-phenoxyessigsäure*

Zu einer Lösung aus 15.9 g (0.1 mol) 4-Hydroxybenzylaminhydrochlorid in 100 ml abs. Pyridin tropft man unter Kühlen 15.7 g (0.2 mol) Acetylchlorid. Anschließend wird 1 Stunde bei 20°C gerührt, dann 15 Minuten lang auf dem siedenden Wasserbad erhitzt, noch warm in Eiswasser gegossen und mit conc. Salzsäure angesäuert. Man extrahiert nun mit Chloroform, trocknet die Chloroformphase mit Natriumsulfat und dampft ein. Das Rohprodukt löst man in Essigester. Nach Zugabe von Ligroin fällt reiner Essigsäure-(4-acetaminomethyl-phenyl)-ester aus. Ausbeute 18.86 g (91% d. Th.), Schmp. 78—79°C.

Man erwärmt ein Gemisch aus 106.8 g (0.515 mol) Essigsäure-(4-acetaminomethyl-phenyl)-ester, 800 ml Methanol und 1030 ml 1 N Kalilauge 2 Stunden auf 50°C, destilliert im Vakuum das Methanol ab und säuert mit Salzsäure an. Nach Einengen der wässrigen Phase wird abgesaugt und mit Ethanol gewaschen. Man erhält so 55.8 g (66% d. Th.) 4-(Acetaminomethyl)-phenol mit dem Schmp. 132—133°C.

Man erhitzt ein Gemisch aus 150.0 g (0.908 mol) 4-(Acetaminomethyl)-phenol, 250.9 g (1.816 mol) wasserfreiem, pulverisiertem Kaliumcarbonat und 3 ltr. 2-Butanon 2 Stunden auf Rückflußtemperatur, kühlt etwas ab und gibt 5 g Kaliumiodid und 244.1 g (1.462 mol) Bromessigsäureethylester zu. Anschließend wird unter Rühren 4 Stunden auf Rückflußtemperatur gehalten, dann kühlt man ab, filtert und wäscht den Filterkuchen mit Aceton. Die vereinigten Filtrate werden im Vakuum eingedampft. Man verreibt den Eindampfrückstand mit Ether, saugt ab und kristallisiert aus Isopropanol um. Ausbeute 211.2 g (92% d. Th.) 4-(Acetaminomethyl)-phenoxyessigsäureethylester mit dem Schmp. 93—94°C.

Ein Gemisch aus 725 ml Ethanol, 125 g (0.5 mol) 4-(Acetaminomethyl)-phenoxyessigsäureethylester, 280 g (5.0 mol) Kaliumhydroxid und 600 ml Wasser wird 14 Stunden auf Rückflußtemperatur gehalten, anschließend abgekühlt und mit conc. Salzsäure auf pH 4 gebracht. Man saugt ab, wäscht den Filterkuchen mit Wasser und kristallisiert aus wässrigem Ethanol um. Es resultieren 73.4 g (81% d. Th.) 4-(Aminomethyl)-phenoxyessigsäure mit dem Schmp. 260°C (Zers.). Das Hydrochlorid hat einen Schmp. von 252—253°C.

Unter Kühlen im Eisbad leitet man auf ein Gemisch aus 89.0 g (0.491 mol) 4-(Aminomethyl)-phenoxyessigsäure und 890 ml abs. Ethanol bis zur Sättigung trocknen Chlorwasserstoff. Anschließend wird 12 Stunden lang bei Raumtemperatur gerührt, wobei langsam eine klare Lösung entsteht, danach im Vakuum eingedampft, wobei 115.3 g (96% d. Th.) 4-Aminomethyl)-phenoxyessigsäureethylester-hydrochlorid zurückbleiben; Schmp. 189—190°C.

Zu einer Lösung aus 24.57 g (0.10 mol) 4-(Aminomethyl)-phenoxyessigsäureethylester-hydrochlorid in 250 ml abs. Pyridin tropft man unter Rühren bei 0°C 35.32 g (0.2 mol) Benzolsulfonylchlorid, rührt anschließend 2 Stunden bei Raumtemperatur und gießt in Eiswasser. Die wässrige Phase wird mit conc. Salzsäure angesäuert, dann mit Ether und Chloroform extrahiert. Die vereinigten Extrakte werden mit verd. Salzsäure gewaschen, getrocknet und im Vakuum eingedampft. Nach Umkristallisieren des Eindampfrückstandes aus Isopropanol erhält man 30.0 g (86% d. Th.) 4-(Benzolsulfonylaminomethyl)-phenoxyessigsäureethylester mit dem Schmp. 110—111°C.

Man mischt 20.96 g (60 mmol) 4-(Benzolsulfonylaminomethyl)-phenoxyessigsäureethylester mit 250 ml Methanol und 180 ml (0.18 mol) 1 N Kalilauge. Die dunkelrote Lösung wird 2 Stunden bei Raumtemperatur gerührt, dann mit Salzsäure angesäuert. Man destilliert nun im Vakuum das Methanol ab und kühlt. Die ausgefallenen Kristalle werden abgesaugt, mit Wasser gewaschen, getrocknet und schließlich aus Isopropanol umkristallisiert. Ausbeute 13.84 g (72% d. Th.) 4-(Benzolsulfonylaminomethyl)-phenoxyessigsäure, Schmp. 142—143°C.

In analoger Weise erhält man durch Umsetzen von 4-Aminophenoxyessigsäureethylester mit den entsprechenden Sulfonylchloriden in Gegenwart von Pyridin:

a) 4-(Benzolsulfonylamino)-phenoxyessigsäureethylester
Schmp. 127—128°C (Essigester); Ausbeute 74% d. Th.
und daraus durch Hydrolyse

4-(Benzolsulfonylamino)-phenoxyessigsäure
Schmp. 157—158°C (Aceton + Wasser); Ausbeute 92% d. Th.

Beispiel 5

*2-{4-[2-(4-Chlorbenzolsulfonylamino)-ethyl]-phenoxy}-2-methyl-propionsäure*

Man erhitzt ein Gemisch aus 44.8 g (0.25 mol) N-Acetyltyramin, 69.5 g (0.5 mol) wasserfreiem, pulverisiertem Kaliumcarbonat und 750 ml absolutem 2-Butanon 2 Stunden unter Rühren auf Rückflußtemperatur, setzt dann 73.2 g (0.375 mol) 2-Brom-2-methyl-propionsäureethylester und 1 g Kaliumiodid zu und erhitzt wiederum auf Rückflußtemperatur.

Nach 40 und 70 Stunden Kochen werden jeweils zusätzliche 35 g Kaliumcarbonat und 36.6 g 2-Brom-2-methyl-propionsäureethylester zugefügt. Nach insgesamt 130 Stunden Umsetzungsdauer engt man im Vakuum ein, gießt auf Eiswasser und extrahiert nun mit Ether. Der Etherextrakt wird dreimal mit 0.5 N Natronlauge, dann mit Wasser gewaschen, schließlich über Calciumchlorid getrocknet und eingedampft. Es bleiben 83.8 g eines öligen Rückstandes, der noch 2-Brom-2-methyl-propionsäure-ethylester enthält. Das Öl wird 5 Stunden im 0.1 Torr-Vakuum auf 70°C gehalten, dann abgekühlt. Der entstandene Kristallbrei wird mit Ligroin gewaschen und getrocknet. Ausbeute 69.8 g (95% d. Th.), Schmp. des noch nicht ganz reinen 2-[4-(2-Acetaminoethyl)-phenoxy]-2-methyl-propionsäureethylesters 51—52°C.

Eine Lösung von 119.1 g (0.407 mol) 2-[4-(2-Acetaminoethyl)-phenoxy]-2-methyl-propionsäure-ethylesters in 750 ml Alkohol wird mit einer Lösung von 224.4 g (4.0 mol) Kaliumhydroxid in 800 ml Wasser gemischt und 8 Stunden auf Rückflußtemperatur erhitzt. Unter Kühlen gibt man exakt 4.0 mol Chlorwasserstoff (z.B. in Form einer 2 N Salzsäure) zu, kühlt stärker ab und saugt nach einiger Zeit die ausgeschiedenen Kristalle ab. Diese werden mit Wasser gewaschen und getrocknet; 48.4 g (53% d. Th.), Schmp. 274°C (Zers.).

Aus der Mutterlauge erhält man nach dem Abdestillieren des Alkohols und Kühlen weitere 32.5 g (36% d. Th.) vom Schmp. 263—270°C. Die rohe 2-[4-(2-Aminoethyl)-phenoxy]-2-methyl-propionsäure wird aus Alkohol + Wasser (4:1 Vol.) umkristallisiert und hat dann einen Schmp. von 284°C. Das Hydrochlorid schmilzt bei 187—189°C.

Man begast eine Lösung von 58 g (0.26 mol) dieser Carbonsäure in 600 ml absolutem Ethanol unter Rühren und Eiskühlung von der Oberfläche her bis zur Sättigung mit trockenem Chlorwasserstoff. Der Ansatz bleibt nun 12 Stunden verschlossen stehen. Anschließend werden im Vakuum Alkohol und Chlorwasserstoff entfernt. Man fügt zum Rückstand Wasser, extrahiert dreimal mit Ether, macht die wässrige Phase deutlich alkalisch und extrahiert sie dreimal mit Chloroform. Der Chloroformextrakt wird mit wenig Wasser gewaschen, über Kaliumcarbonat getrocknet und eingedampft. Durch Destillation des Rückstandes erhält man zwischen 125 und 128°C/0.1 Torr 53.2 g (82% d. Th.) farblosen 2-[4-(2-Aminoethyl)-phenoxy]-2-methyl-propionsäureethylester. Das gaschromatographisch reine Produkt hat den Brechungsindex $n_D^{20} = 1.5075$.

Durch Umsetzen von 2-[4-(2-Aminoethyl-phenoxy]-2-methyl-propionsäureethylester mit 4-Chlorbenzolsulfonylchlorid in Analogie zu Beispiel 2 erhält man mit einer Ausbeute von 69% d. Th. farblosen 2-{4-[2-(4-Chlorbenzolsulfonylamino)-ethyl]-phenoxy}-2-methyl-propionsäureethylester als viskoses Öl,

und daraus durch Hydrolyse

2-{-[2-(4-Chlorobenzolsulfonanylamino)-ethyl]-phenoxy}-2-methyl-propionsäure
Ausbeute 67% d. Th., Schmp. 116°C (Aceton).
In Analogie dazu erhält man

2-{4-[2-(Benzolsulfonylamino)-ethyl]-phenoxy}-2-methyl-propionsäureethylester
aus

2-[4-(2-Aminoethyl)-phenoxy-2-methyl-propionsäureethylester und Benzolsulfonylchlorid
Schmp. 66—67°C (Isopropanol + Ligroin); Ausbeute 71% d. Th.
und daraus durch Hydrolyse

2-{4-[2-(Benzolsulfonylamino)-ethyl]-phenoxy}-2-methyl-propionsäure
Schmp. 128—129°C (Essigester + Ligroin); Ausbeute 85% d. Th.

Beispiel 6

*4-[2-(Benzolsulfonylamino)-ethyl]-phenoxyacetamid*

Man erhitzt ein Gemisch aus 67 g (0.2 mol) 4-[2-(Benzolsulfonylamino)-ethyl]-phenoxyessigsäure, 400 ml Benzol und 71.5 g (0.6 mol) Thionylchlorid 4 Stunden auf Rückflußtemperatur. Dann

werden Benzol und überschüssiges Thionylchlorid im Vakuum abdestilliert. Rohausbeute: quantitativ. Nach Umkristallisieren aus Methylenchlorid erhält man 61 g (86% d. Th.) 4-[2-(Benzolsulfonylamino)-ethyl]-phenoxyacetylchlorid mit dem Schmp. 78.5—79°C. Man kann hier mit gleichem Erfolg auch ohne Benzolzusatz, also mit reinem Thionylchlorid, arbeiten.

Man versetzt unter Rühren eine Lösung aus 10.6 g (30 mmol) 4-[2-(Benzolsulfonylamino)-ethyl]-phenoxyacetylchlorid und 100 ml abs. Dioxan bei Raumtemperatur mit 3.3 ml konzentrierter Ammoniaklösung, erwärmt kurz auf 40°C und kühlt ab. Der Kolbeninhalt wird dann auf Eiswasser gegossen. Man saugt ab, wäscht den Filterkuchen mit Wasser und digeriert mit Natriumhydrogencarbonatlösung. Nach Absaugen und Waschen mit Wasser wird aus Aceton umkristallisiert. Ausbeute 7.2 g (72% d. Th.) 4-[2-(Benzolsulfonylamino)-ethyl]-phenoxyacetamid, Schmp. 118—119°C.

Durch Umsetzen von 4-[2-(Benzolsulfonylamino)-ethyl]-phenoxyacetylchlorid mit den entsprechenden Aminen erhält man die folgenden Amide:

a) 4-[2-(Benzolsulfonylamino)-ethyl]-phenoxyacetanilid

Zur einer Lösung aus 7.08 g (20 mmol) Säurechlorid und 35 ml abs. Benzol tropft man unter Rühren 3.73 g (40 mmol) Anilin, erwärmt kurz auf 40°C und destilliert nun das Benzol ab. Zum Rückstand gibt man Methylenchlorid und verd. Salzsäure, schüttelt kräftig durch und trennt die Phasen. Die Methylenchloridphase wird mit 2 N Salzsäure und Wasser gewaschen, getrocknet und eingedampft. Nach dem Umkristallisieren aus Essigester erhält man 5.58 g (68% d. Th.) Produkt mit dem Schmp. 123°C.

b) 4-[2-(Benzolsulfonylamino)-ethyl]-phenoxyessigsäure-(2-ethoxycarbonyl-ethylamid)

Zu einem Gemisch aus 4.6 g (30 mmol) β-Aminopropionsäureethylester-hydrochlorid, 4.1 g (30 mmol) pulverisiertem Kaliumcarbonat und 100 ml abs. Pyridin tropft man nach 20 Minuten Rühren unter Eiskühlung 10.6 g (30 mmol) Säurechlorid. Anschließend läßt man auf 20°C kommen, erwärmt dann 30 Minuten auf 50°C, kühlt ab und gießt in ca. 500 ml Eiswasser. Durch Zugabe von Salzsäure bringt man auf pH 5.5, dann extrahiert man mit Methylenchlorid. Die Methylenchloridphase wird mehrmals mit verd. Salzsäure, dann mit Natriumhydrogencarbonatlösung und Wasser gewaschen, mit Natriumsulfat getrocknet und eingedampft. Es bleiben 10.5 g (81% d. Th.) öliges 4-[2-(Benzolsulfonylamino)-ethyl]-phenoxyessigsäure-(2-ethoxycarbonyl-ethylamid) mit dem Brechungsindex $n_D^{20} = 1.5490$ zurück.

Daraus erhält man durch Hydrolyse in Analogie zu Beispiel 2

4-[2-(Benzolsulfonylamino)-ethyl]-phenoxyessigsäure-(2-carboxyethylamid)
Schmp. 64—65°C (Aceton + Wasser); Ausbeute 86% d. Th.

c) 4-[2-(Benzolsulfonylamino)-ethyl]-phenoxyessigsäure-(4-ethoxycarbonyl-anilid)
in Analogie zu b) aus dem Säurechlorid und 4-Aminobenzoesäureethylester-hydrochlorid, Schmp. 157—158°C (Essigester); Ausbeute 72% d. Th.
und daraus durch Hydrolyse

4-[2-(Benzolsulfonylamino)-ethyl]-phenoxyessigsäure-(4-carboxyanilid)
Schmp. 185—186°C (Aceton + Wasser); Ausbeute 65% d. Th.

d) 4-[2-(Benzolsulfonylamino)-ethyl]-phenoxyessigsäure-[4-(ethoxycarbonylmethylenoxy)-phenethylamid]
in Analogie zu b) aus dem Säurechlorid und 4-(2-Aminoethyl)-phenoxyessigsäureethylester.
Schmp. 97—98°C (Essigester); Ausbeute 68% d. Th.
und daraus durch Hydrolyse

4-[2-(Benzolsulfonylamino)-ethyl]-phenoxyessigsäure-[4-(carboxymethylenoxy)-phenethylamid]
Schmp. 148—149°C (Wasser + Aceton); Ausbeute 60% d. Th.

## Beispiel 7

*4-[2-(n-Octansulfonylamino)-ethyl]-phenoxyessigsaeure*

Man suspendiert 26.1 g (0.1 mol) 4-(2-Aminoethyl)-phenoxyessigsaeure-ethylester-hydrochlorid und 21.3 g (0.1 mol) n-Octansulfonylchlorid in 400 ml Benzol, tropft unter sehr heftigem Ruehren eine Loesung aus 27.6 g (0.2 mol) Kaliumcarbonat und 400 ml Wasser zu, ruehrt 10 min nach und trennt dann die Phasen. Die Benzolphase wird mit Wasser gewaschen, getrocknet ($Na_2SO_4$) und i.Vak. eingedampft. Nach dem Umkristallisieren aus Essigester erhaelt man 32.6 g (82% d. Th.) 4-[2-(n-Octansulfonylamino)-ethyl]-phenoxyessigsaeure-ethylester mit dem Schmp. 65—66°C (Essigester).

Ein Gemisch aus 15.4 g (38 mmol) Ethylester, 38 ml 2 N—KOH und 38 ml Ethanol wird 3 h bei 40°C gehalten. Dann destilliert man i.Vak. das Ethanol ab, saeuert mit HCl an, saugt ab und kristallisiert aus einem Wasser-Dioxan-Gemisch um. Es resultieren 12.4 g (88% d. Th.) 4-[2-(n-Octansulfonylamino)-ethyl]-phenoxyessigsaeure mit dem Schmp. 128—129°C.

Beispiel 8.

*4-[2-(n-Hexadecansulfonylamino)-ethyl]-phenoxyessigsaeure*

Zu einem Gemisch aus 4.4 g (16.9 mmol) 4-(2-Aminoethyl)-phenoxyessigsaeure-ethylester-hydrochlorid, 50 ml Benzol und 6.8 g (68 mmol) Triethylamin tropft man bei 0°C unter heftigem Ruehren 5.5 g (16.9 mmol) n-Hexadecansulfonylchlorid, ruehrt weitere 2 h im Eisbad und laesst ueber Nacht bei 20°C stehen. Dann wird auf Eiswasser gegossen, mit Salzsaeure sauer gestellt und mit Ether extrahiert. Man waescht die Etherphase mit Wasser, trocknet ($Na_2SO_4$), dampft ein und kristallisiert den Rueckstand aus Ethanol um. Es resultieren 5.8 g (67% d. Th.) 4-[2-(n-Hexadecansulfonylamino)-ethyl]-phenoxyessigsaeure-ethylester mit dem Schmp. 91—92°C.

Die Hydrolyse des Esters erfolgte in Analogie zu Beispiel 7 mittels waessriger Kalilauge in Methanol. Ausbeute: 68% d. Th. 4-[2-(n-Hexadecansulfonylamino)-ethyl]-phenoxyessigsaeure mit dem Schmp. 137.5—138°C (Ethanol).

Beispiel 9

In zu Beispiel 3 analoger Weise erhaelt man durch Umsetzen von 4-(2-Aminoethyl)-phenoxyessigsaeure-ethylester-hydrochlorid mit den entsprechenden Sulfonylchloriden in Gegenwart von Pyridin und Kaliumcarbonat:

a) 4-[2-(2-Phenyl-ethansulfonylamino)-ethyl]-phenoxyessigsaeure-ethylester
als viskoses Oel (Rohprodukt); Ausbeute 76% d. Th.
und daraus durch Hydrolyse
4-[2-(2-Phenyl-ethansulfonylamino)-ethyl]-phenoxyessigsaeure
Schmp. 124—125°C (Ethanol + 10% Wasser); Ausbeute 74% d. Th.

b) 4-{2-[2-(4-Chlorphenyl)-ethansulfonylamino]-ethyl}-phenoxyessigsaeure-ethylester
als viskoses Oel (Rohprodukt); Ausbeute 57% d. Th.
und daraus durch Hydrolyse

4-{2-[2-(4-Chlorphenyl)-ethansulfonylamino]-ethyl}-phenoxyessigsaeure
Schmp. 128—130°C (Essigester + Ligroin); Ausbeute 73% d. Th.

c) 4-[2-(4-Acetylbenzolsulfonylamino)-ethyl]-phenoxyessigsaeure-ethylester
Schmp. 113.5—114°C (Essigester + Ligroin); Ausbeute 52% d. Th.
und daraus durch Hydrolyse

4-[2-(4-Acetylbenzolsulfonylamino)-ethyl]-phenoxyessigsaeure
Schmp. 162°C (Aceton); Ausbeute 63% d. Th.

Beispiel 10

*4-[2-(Benzolsulfonylamino)-ethyl]-phenoxyessigsaeure-(1-hydroxy-2-propylamid)*

Zu einem Gemisch aus 5.4 g (72 mmol) 1-Hydroxy-2-amino-propan und 35 ml Wasser tropft man waehrend 2 h bei 0—5°C eine Loesung aus 6.4 g (18 mmol) 4-[2-(Benzolsulfonylamino)-ethyl]-phenoxy-acetylchlorid und 25 ml abs. Benzol, wobei sich langsam ein Niederschlag bildet. Nach Stehen ueber Nacht bei 20°C saugt man ab, waeschte den Filterkuchen mit verd. NaOH, dann mit Wasser und trocknet. Nach Umkristallisieren aus Essigester erhaelt man 5.2 g (74% d. Th.) mit dem Schmp. 90—91°C.

Beispiel 11

*4-[2-(N-Methyl-benzolsulfonylamino)-ethyl]-phenoxyessigsaeure*

Zu einem Gemisch aus 9.1 g (25 mmol) 4-[2-(Benzolsulfonylamino)-ethyl]-phenoxyessigsaeure-ethylester, 50 ml Hexamethylphosphorsaeure-triamid und 50 ml abs. Toluol gibt man 0.6 g (25 mmol) Natriumhydrid (als Mineraloel-Suspension) und ruehrt anschliessend 2 h bei 80°C. Dann wird abgekuehlt, mit einem Gemisch aus 10.6 g (75 mmol) Methyliodid und 10 ml Hexamethylphosphorsaeure-triamid versetzt, 15 min bei 20°C geruehrt und dann 3 h bei 80°C gehalten. Nach dem Abkuehlen giesst man auf Eiswasser, bringt mittels HCl auf pH 3 und extrahiert mehrmals mittels Toluol. Die Toluolphase wird eingedampft, der Eindampfrueckstand mittels Kieselgel/Toluol chromatographiert. Man erhaelt 3.9 g (41% d. Th.) reinen 4-[2-(N-Methyl-benzolsulfonylamino)-ethyl]-phenoxyessigsaeureethylester als farbloses Oel mit dem Brechungsindex $n_D^{20} = 1.5440$.

Diese Verbindung wird auch auf folgendem Wege erhalten:

Man loest 9.25 g (54 mmol) Benzolsulfonsaeuremonomethylamid in 75 ml Hexamethylphosphorsaeure-triamid, gibt 1.3 g (54 mmol) Natriumhydrid (in Form einer Mineraloel-Suspension) zu, ruehrt 20 min bei 20°C und fuegt nun 14.9 g (54 mmol) 4-(2-Bromethyl)-phenoxyessigsaeure-ethylester zu. Man ruehrt 24 h bei 50°C, kuehlt ab und giesst auf Eiswasser. Mittels verd. HCl wird auf pH 3 gebracht, dann mehrmals mit Toluol extrahiert. Die Toluolphase wird mit Wasser gewaschen und mittels Natriumsulfat getrocknet. Man chromatographiert nun an einer Kieselgel-Saeule/Toluol und erhaelt 9.2 g (45% d. Th.) des gewuenschten Esters.

Daraus erhaelt man durch Hydrolyse mittels 1 N—KOH in Ethanol:

4-[2-(N-Methyl-benzolsulfonylamino)-ethyl]-phenoxyessigsaeure
Schmp. 103—104°C (Essigester + Ligroin); Ausbeute 91% d. Th.

Beispiel 12

*4-[3-(Benzolsulfonylamino)-propyl]-phenoxyessigsaeure*

Man haelt ein Gemisch aus 14.5 g (75 mmol) 4-(3-Acetamino-propyl)-phenol, 16.8 g (122.5 mmol) pulv. Kaliumcarbonat und 150 ml Butanon-2 2 h auf Rueckflusstemperatur, gibt dann 20.6 g (122.5 mmol) Bromessigsaeure-ethylester und eine Spatelspitze Kaliumiodid zu und haelt 6 h bei 80°C. Dann wird abgesaugt und das Filtrat im Vak. eingedampft, zur Abtrennung von ueberschuessigem Bromessigester zum Schluss im Oelpumpenvakuum. Man nimmt den Eindampfrueckstand in Methylenchlorid auf und extrahiert mit kalter 0.5 N—NaOH, dann mit Wasser. Nach Trocknen mittels $Na_2SO_4$ wird eingedampft und durch Abkuehlen unter Etherzusatz zur Kristallisation gebracht. Nach Umkristallisieren aus Ether erhaelt man 14.9 g (71% d. Th.) 4-(3-Acetamino-propyl)-phenoxyessigsaeure-ethylester mit dem Schmp. 57—58°C.

Ein Gemisch aus 16.2 g (58 mmol) 4-(3-Acetamino-propyl)-phenoxyessigsaeure-ethylester, 100 ml Ethanol, 100 ml Wasser und 32.5 g (0.58 mol) Kaliumhydroxid wird 16 h auf Rueckflusstemperatur gehalten. Dann kuehlt man ab, bringt mittels 6 N—HCl auf pH 6.5 und saugt den entstehenden sandfarbenen Niederschlag ab. Nach Waschen mit Wasser und Trocknen erhaelt man 12.0 g (quant. Ausb.) 4-(3-Aminopropyl)-phenoxyessigsaeure mit dem Schmp. 248°C.

Man erwaermt ein Gemisch aus 9.7 g (46.5 mmol) 4-(3-Aminopropyl)-phenoxyessigsaeure, 6.9 g (50 mmol) Kaliumcarbonat und 100 ml Wasser auf 80°C und tropft bei dieser Temperatur 8.5 g (48 mmol) Benzolsulfochlorid zu. Danach wird weitere 2 h bei 80°C gehalten, dann abgekuehlt und mittels 2 N—HCl auf pH 2 gebracht. Man saugt den ausfallenden Niederschlag ab, trocknet ihn und kristallisiert aus Essigester, dann aus Eisessig um und erhaelt 8.26 g (51% d. Th.) 4-[3-(Benzolsulfonyl)-propyl]-phenoxyessigsaeure mit dem Schmp. 146—147°C.

Beispiel 13

*2-{4-[2-(Benzolsulfonylamino)-ethyl]-phenoxy}-2-methyl-propionsaeure*
(nach der "Chloroform-Aceton-Method")

Zu einem Gemisch aus 4.45 g (16.1 mmol) 4-[2-(Benzolsulfonylamino)-ethyl]-phenol und 77 ml Aceton gibt man bei 20°C unter Ruehren 9.4 g (235 mmol) Natriumhydroxid und tropft nun innerhalb 2 h 8.7 g (72.9 mmol) Chloroform zu, wobei durch zeitweiliges Kuehlen die Innentemperatur bei 30—35°C gehalten wird. Dann wird 30 min bei 30°C nachgeruehrt, darauf 2.5 h auf Rueckflusstemperatur gehalten. Man dampft dann das Aceton i.Vak. ab, nimmt den Rueckstand in Wasser auf und extrahiert mit Ether. Die waessrige Phase wird angesaeuert und mit Essigester extrahiert. Die Essigesterphase trocknet man ($Na_2SO_4$) und dampft i.Vak. ein, versetzt den Rueckstand mit 50 ml Wasser und 5 g Natriumhydrogencarbonat und ruehrt, wobei eine klare Loesung entsteht. Diese wird wiederum mit Ether extrahiert. Die waessrige Phase saeuert man nun an und extrahiert mit Essigester. Nach Eindampfen des Essigesters kristallisiert man aus einem Essigester-Ligroin-Gemisch unter Kohlezusatz um und erhaelt 2.64 g (45% d. Th.) der Saeure, die in allen physikalischen Eigenschaften mit der unter Beispiel 5 genannten Substanz identisch ist.

Beispiel 14

*2-[4-(4-Chlorbenzolsulfonylamino)-phenoxy]-2-methyl-propionsaeure*

Man haelt unter Ruehren ein Gemisch aus 139.1 g (1 mol) 4-Nitrophenol, 1 Liter Butanon-2 und 207.3 g (1.5 mol) pulv., wasserfreiem Kaliumcarbonat 2 h auf Rueckflusstemperatur, gibt nun 292.5 g (1.5 mol) 2-Brom-2-methyl-propionsaeureethylester zu und haelt nun weitere 92 h auf Rueckflusstemperatur. Nach Zugabe von weiteren 69.1 g (0.5 mol) Kaliumcarbonat und 97.5 g (0.5 mol) 2-Brom-2-methyl-propionsaeure-ethylester wird weitere 20 h bei Rueckflusstemperatur geruehrt. Dann saugt man ab, dampft das Filtrat i.Vak. ein (zur Beseitigung von ueberschuessigem Bromester zum Schluss im Hochvakuum), nimmt den Eindampfrueckstand in Ether auf, filtriert und extrahiert nun die Etherphase mehrfach mit 1 N—NaOH. Nach dem Neutralwaschen und Trocknen wird eingedampft; wobei 153 g (60% d. Th.) oeliges Rohprodukt zurueckbleiben. Nach Destillation bei 155.5—156°C/0.05 Torr erhaelt man in 46% Ausbeute reinen 2-Methyl-2-(4-nitrophenoxy)-propionsaeure-ethylester als gelbes Oel mit dem Brechungsindex $n_D^{20} = 1.5288$.

Ein Gemisch aus 50.6 g (0.2 mol) 2-Methyl-2-(4-nitrophenoxy)-propionsaeure-ethylester, 500 ml Ethanol und ca. 20 g 5-proz. Palladiumkohle wird in der Schuettelente bei Raumtemperatur und Normaldruck hydriert, bis die erforderliche Menge Wasserstoff aufgenommen ist. Anschliessend saugt man ab, dampft die fluessige Phase ein und erhaelt in quantitativer Ausbeute rohen 2-(4-Aminophenoxy)-2-methyl-propionsaeure-ethylester als destillierbares Oel (Sdp. 134—135°C/0.05 Torr); $n_D^{20} = 1.5034$; Schmp. des Hydrochlorids: 153—154°C.

Zu einem Gemisch aus 13.0 g (50 mmol) 2-(4-Aminophenoxy)-2-methyl-propionsaeure-ethylester-hydrochlorid un 200 ml abs. Pyridin gibt man innerhalb 30 min bei 0—5°C 10.6 g (50 mmol) 4-Chlor-benzolsulfochlorid, ruehrt 1 h bei 5°C nach und haelt schliesslich 2 h bei 80°C. Dann kuehlt man, ruehrt in Eiswasser ein und saeuert mittels HCl auf pH 5.5 an, wobei sich ein oeliges Produkt abscheidet. Dieses wird in Ether aufgenommen. Man waescht die Etherphase nacheinander mit 6 N—HCl, Wasser, $NaHCO_3$-Loesung und wiederum mit Wasser, trocknet und dampft i.Vak. ein. Es bleiben 18.8 g (95% d. Th.) roher 2-[4-(4-Chlorbenzolsulfonylamino)-phenoxy]-2-methyl-propionsaeure-ethylester als farbloses Oel zurueck. Durch Verseifen des Rohesters mittels 1 N-Kalilauge in Ethanol in Analogie zu Beispiel 2 erhaelt man in 91% Ausbeute kristalline 2-[4-(4-Chlorbenzolsulfonylamino)-phenoxy]-2-methyl-propionsaeure mit dem Schmp. 146.5—147°C (Essigester + Ligroin).

In analoger Weise erhaelt man

a) 2-[4-(4-Chlorstyrolsulfonylamino)-phenoxy]-2-methyl-propionsaeure-ethylester
aus p-Chlorstyrol-sulfonylchlorid und 2-(4-Aminophenoxy)-2-methyl-propionsaeure-ethylester
Schmp. 130—131°C (Essigester); Ausbeute 87% d. Th.
und daraus durch Hydrolyse

2-[4-(4-Chlorstyrolsulfonylamino)-phenoxy]-2-methyl-propionsaeure
Schmp. 172—173°C (Essigester); Ausbeute 73% d. Th.

b) 2-{4-[2-(4-Chlorstyrolsulfonylamino)-ethyl]-phenoxy}-2-methyl-propionsaeure-ethylester
aus p-Chlorstyrol-sulfonylchlorid in 2-[4-(2-Aminoethyl)-phenoxy]-2-methyl-propionsaeure-ethylester
Farbloses Oel, $n_D^{20} = 1.5625$
und daraus durch Hydrolyse

2-{4-[2-(4-Chlorstyrolsulfonylamino)-ethyl]-phenoxy}-2-methyl-propionsaeure
Schmp. 148—149°C (Benzol); Ausbeute 76% d. Th.

c) 2-{4-{2-[2-(4-Chlorphenyl)-ethansulfonylamino]-ethyl}-phenoxy}-2-methyl-propionsaeure-ethylester
aus 2-(4-Chlorphenyl)-ethansulfonylchlorid und 2-[4-(2-Aminoethyl)-phenoxy]-2-methyl-propionsaeure-ethylester,
Farbloses Oel; Ausbeute 77% d. Th.
und daraus durch Hydrolyse

2-{4-{2-[2-(4-Chlorphenyl)-ethansulfonylamino]-ethyl}-phenoxy}-2-methyl-propionsaeure
Schmp. 138—139°C (Essigester + Ligroin); Ausbeute 53% d. Th.

## Beispiel 15

*2-{4-[2-(4-Chlorbenzolsulfonylamino)-ethyl]-phenoxy}-n-hexansaeure*

Man ruehrt ein Gemisch aus 17.92 g (0.1 mol) 4-(2-Acetaminoethyl)-phenol, 17.3 g (0.125 mol) pulv. Kaliumcarbonat und 150 ml Butanon-2 2 h bei Rueckflusstemperatur, gibt dann 27.9 g (0.125 mol) 2-Bromhexansaeure-ethylester und eine Spatelspitze Kaliumiodid zu und haelt weitere 28 h auf Rueckflusstemperatur. Dann wird abgesaugt und das Filtrat i.Vak. eingedampft. Man nimmt in Ether auf, extrahiert die Etherphase mit 2 N—HCl, Wasser, 0.5 N—NaOH und wiederum mit Wasser, trocknet ueber $Na_2SO_4$ und dampft ein. Es resultieren 32.1 g (quant. Ausbeute) 2-[4-(2-Acetaminoethyl)-phenoxy]-n-hexansaeure-ethylester als farbl. Oel mit dem Brechungsindex $n_D^{20} = 1.5010$.

Man haelt ein Gemisch aus 25.7 g (80 mmol) 2-[4-(2-Acetaminoethyl)-phenoxy]-n-hexansaeure-ethylester, 150 ml Wasser, 150 ml Ethanol und 44.9 g (0.8 mol) Kaliumhydroxid 16 h auf Rueckflusstemperatur und destilliert anschliessend i.Vak. den Alkohol ab. Nach Zugabe von 150 ml Wasser wird mit Salzsaeure bis pH 4 angesaeuert und der entstandene Niederschlag abgesaugt, mit Wasser gewaschen und getrocknet. Es resultieren 12.5 g (62% d. Th.) 2-[4-(2-Aminoethyl)-phenoxy]-n-pentancarbonsaeure mit dem Schmp. 274—276°C.

Durch Umsetzen der 2-[4-(2-Aminoethyl)-phenoxy]-n-pentancarbonsaeure mit 4-Chlor-benzolsulfonylchlorid in Gegenwart von waessr. Kaliumcarbonatloesung in Analogie zu Beispiel 1 erhaelt man in 32% Ausbeute die 2-{4-[2-(4-Chlorbenzolsulfonylamino)-ethyl]-phenoxy}-n-pentancarbonsaeure mit dem Schmp. 138—139°C (Essigester, Cyclohexan).

**Patentansprüche**

1. Phenoxyalkylcarbonsäure-Derivate der Formel I

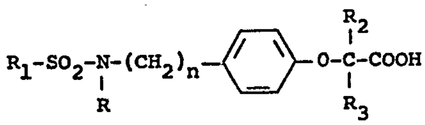

(I),

in welcher
R Wasserstoff oder eine niedere Alkylgruppe,
$R_1$ eine Alkylgruppe oder eine Aryl-, Aralkyl- oder Aralkenylgruppe, deren Aryl-Rest jeweils ein- oder mehrfach durch Hydroxyl, Halogen, Trifluormethyl oder niedere Alkyl-, Alkoxy- oder Acylgruppen substituiert sein kann,
$R_2$ und $R_3$, die gleich oder verschieden sein können, Wasserstoff oder eine niedere Alkylgruppe, und
n die Zahlen 0 bis 3
bedeuten, sowie deren physiologisch unbedenklichen Salze, Ester und Amide.

2. Verfahren zur Herstellung von Phenoxyalkylcarbonsäure-Derivaten der Formel I

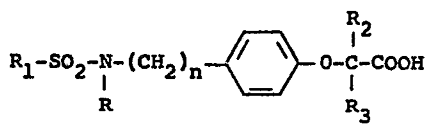

(I),

in welcher
R Wasserstoff oder eine niedere Alkylgruppe,
$R_1$ eine Alkylgruppe oder eine Aryl-, Aralkyl- oder Aralkenylgruppe, deren Aryl-Rest jeweils ein- oder mehrfach durch Hydroxyl, Halogen, Trifluormethyl oder niedere Alkyl, Alkoxy- oder Acylgruppen substituiert sein kann,
$R_2$ und $R_3$, die gleich oder verschieden sein können, Wasserstoff oder eine niedere Alkylgruppe, und
n die Zahlen 0 bis 3
bedeuten, sowie deren physiologisch unbedenklichen Salze, Ester und Amide, dadurch gekennzeichnet, daß man
a) ein Amin der Formel II

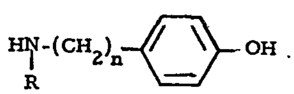

(II),

in welcher
R und n die oben angegebene Bedeutung haben, gegebenenfalls unter intermediärem Schutz der Amino- bzw. Hydroxylgruppe in an sich bekannter Weise in beliebiger Reihenfolge mit einer Sulfonsäure der Formel III

$$R_1—SO_2OH \quad (III),$$

in welcher
$R_1$ die oben angegebene Bedeutung hat, bzw. einem Säure-Derivat derselben und mit einer Verbindung der Formel IV

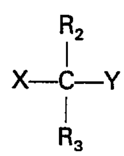

(IV),

in welcher
$R_2$ und $R_3$ die oben angegebene Bedeutung haben, X eine Reaktive Gruppe und Y die Gruppe $—COOR_4$, in der $R_4$ ein Wasserstoffatom oder eine niedere Alkylgruppe bedeutet, eine Säureamidgruppe oder einen Rest darstellt, der nach erfolgter Kondensation in die $COOR_4$-Gruppe oder in eine Säureamidgruppe überführt wird,
oder für den Fall, daß $R_2$ und $R_3$ niedere Alkylgruppen bedeuten, mit einem Gemisch aus einem aliphatischen Keton, Chloroform und einem Alkalihydroxid umsetzt,
oder

b) ein Sulfonamid der Formel V

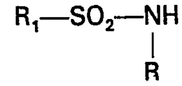

(V),

in welcher
R und $R_1$ die oben angegebene Bedeutung haben, mit einer Verbindung der Formel VI

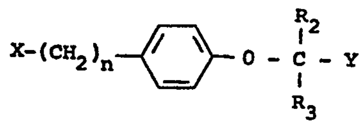

(VI),

in welcher
X, n, $R_2$, $R_3$ und Y die oben angegebene Bedeutung haben,
umsetzt,
worauf man gegebenenfalls Verbindungen der Formal I mit R=Wasserstoff durch bekannte Verfahren N-alkyliert, oder gegebenenfalls die erhaltenen Säurederivate der Formel I in die freie Säure oder gewünschtenfalls die erhaltene freie Säure der Formel I in ein physiologisch unbedenkliches Salz, einen solchen Ester oder ein solches Amid überführt.

3. Arzneimittel mit pharmakologisch unbedenklichen Träger- und Hilfsstoffen, dadurch gekennzeichnet, daß es eine Verbindung gemäß Anspruch 1 enthält.

4. Verwendung der Verbindungen gemäß Anspruch 1 zur Herstellung von Arzneimitteln mit thrombozythen-aggregation-hemmender und/oder lipidsenkender Wirkung.

**Revendications**

1. Dérivés de l'acide phénoxyalkylcarboxylique de formule I

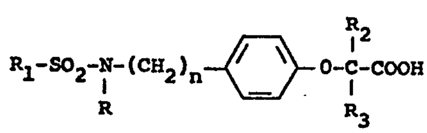

(I)

dans laquelle:
R est un atome d'hydrogène ou un groupe alkyle inférieur,
$R_1$ est un groupe alkyle ou un groupe aryle, aralkyle ou aralcényle, dont le reste aryle porte chaque fois un ou plusieurs groupes hydroxyle, trifluorométhyle, alkyle, alcoxyle ou acyle, ou un ou plusieures atomes d'halogène,
$R_2$ et $R_3$ qui peuvent être identiques ou différents sont des atomes d'hydrogène ou des groupes alkyle inférieurs, et
n est un nombre compris entre 0 et 3,
ainsi que leurs sels, esters et amides physiologiquement inoffensifs.

2. Procédé pour la fabrication de dérivés de l'acide phénoxyalkylcarboxylique de formule I

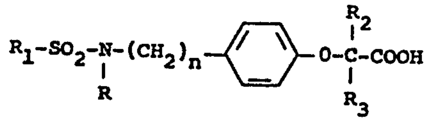

(I)

dans laquelle:
R est un atome d'hydrogène ou un groupe alkyle inférieur,
$R_1$ est un groupe alkyle ou un groupe aryle, aralkyle ou aralcényle, dont le reste aryle porte chaque fois un ou plusieurs groupes hydroxyle, trifluorométhyle, alkyle, alcoxyle ou acyle, ou un ou plusieurs atomes d'halogène,
$R_2$ et $R_3$ qui peuvent être identiques ou différents sont des atomes d'hydrogène ou des groupes alkyle inférieurs, et
n est un nombre compris entre 0 et 3.
ainsi que leurs sels, esters et amides physiologiquement inoffensifs, caractérisé en ce que l'on fait réagir:
a) une amine de formule II

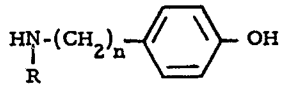

(II)

dans laquelle:
R et n ont la signification ci-dessus indiquée,
éventuellement avec protection intermédiaire du groupe amino ou hydroxyle, de façon en soi connue dans un ordre quelconque avec un acide sulfurique de formule III

$$R_1\text{—}SO_2OH \qquad \text{(III)}$$

dans laquelle:
$R_1$ a la signification ci-dessus indiquée, ou avec und dérivé acide de cet acide (III), et avec un composé de formule IV

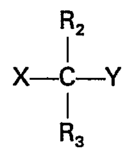

(IV)

dans laquelle:
$R_2$ et $R_3$ ont la signification ci-dessus indiquée, X est un groupe réactif et Y le groupe —$COOR_4$, dans lequel $R_4$ est un atome d'hydrogène ou un groupe alkyle inférieur, un groupe amide d'acide ou un reste qui est transformé après l'achèvement de la condensation en groupe $COOR_4$ ou en un groupe amide d'acide, ou,
dans le cas où $R_2$ et $R_3$ sont des groupes alkyle inférieurs avec un mélange d'une cétone aliphatique, de chloroforme et d'un hydroxyde alcalin,
ou,
b) un sulfonamide de formule V

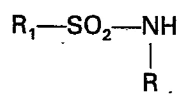

(V)

dans laquelle:
R et $R_1$ ont la signification ci-dessus indiquée, avec un composé de formule VI

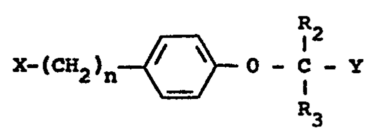

(VI)

dans laquelle:
X, n, $R_2$, $R_3$ et Y ont la signification ci-dessus indiquée, et en ce qu'ensuite on transforme éventuellement les composés de formule I avec R=H par N-alkylation de façon connue, ou bien l'on transforme éventuellement les dérivés acides de formule I en acide libre de formule I ou encore si l'on désire, on transforme l'acide de formule I obtenu en un sel, un ester ou un amide physiologiquement inoffensif.

3. Médicament contenant des substances auxiliaires et de support, caractérisé en ce qu'il contient un composé selon la revendication 1.

4. Utilisation des composés selon la revendication 1 pour la préparation de médicaments ayant une action inhibitrice de l'agrégation des thrombocytes et/ou réductrice de la teneur en lipides.

**Claims**

1. Phenoxyalkylcarboxylic acid derivatives of the formula I:

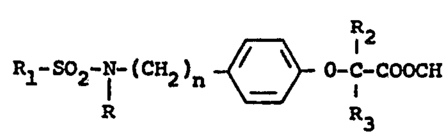

(I),

in which R signifies hydrogen or a lower alkyl radical, $R_1$ an alkyl radical or an aryl, aralkyl or aralkenyl radical, the aryl residue of which can be substituted one or more times by hydroxyl, halogen, trifluoromethyl or lower alkyl, alkoxy or acyl radicals, $R_2$ and $R_3$, which can be the same or different,

hydrogen or a lower alkyl radical and *n* the numbers 0 to 3; as well as the physiologically acceptable salts, esters and amides thereof.

2. Process for the preparation of phenoxyalkylcarboxylic acid derivatives of the formula I:

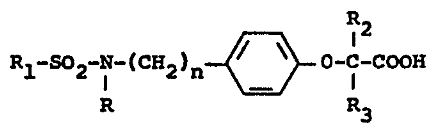

(I),

in which R signifies hydrogen or a lower alkyl radical, $R_1$ an alkyl radical or an aryl, aralkyl or aralkenyl radical, the aryl residue of which can be substituted one or more times by hydroxyl, halogen, trifluoromethyl or lower alkyl, alkoxy or acyl radicals, $R_2$ and $R_3$, which can be the same or different, hydrogen or a lower alkyl radical and *n* the numbers 0 to 3; as well as the physiologically acceptable salts, esters and amides thereof, characterised in that one

a) reacts an amine of the formula II:

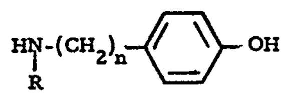

(II),

in which R and *n* have the above-given meaning, optionally with intermediate protection of the amino or hydroxyl group, in per se known manner in any sequence with a sulphonic acid of the formula III:

$$R_1—SO_2OH \qquad (III),$$

in which $R_1$ has the above-given meaning, or with an acid derivative thereof, and with a compound of the formula IV:

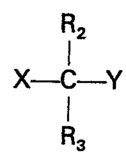

(IV),

in which $R_2$ and $R_3$ have the above-given meaning, X signifies a reactive group and Y the group $—COOR_4$, in which $R_4$ signifies a hydrogen atom or a lower alkyl radical, an acid amide group or a residue which, after condensation has taken place, is converted into the $—COOR_4$ group or into an acid amide group, or, when $R_2$ and $R_3$ signify lower alkyl radicals, is reacted with an aliphatic ketone, chloroform and an alkali metal hydroxide; or

b) reacts a sulphonamide of the formula V:

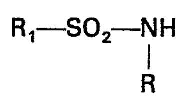

(V),

in which R and $R_1$ have the above-given meaning, with a compound of the formula VI:

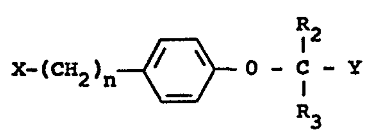

(VI),

in which X, *n*, $R_2$, $R_3$ and Y have the above-given meaning, whereafter one possibly N-alkylates compounds of the formula I with R = hydrogen by known processes or possibly converts the acid derivative obtained of the formula I into the free acid or, if desired, converts the free acid obtained of the formula I into a physiologically acceptable salt, an ester thereof or an amide thereof.

3. Medicament with pharmacologically acceptable carrier and adjuvant materials, characterised in that it contains a compound according to claim 1.

4. Use of compounds according to claim 1 for the preparation of medicaments with thrombocyte aggregation-inhibiting and/or lipid sinking action.